# EUROPEAN PATENT APPLICATION

(11) **EP 4 289 828 A1**
(43) Date of publication of application: **13.12.2023**
(21) Application number: 22178272.5
(22) Date of filing: 10.06.2022
(51) Int. Cl.: C07D 273/04

(54) **METHOD FOR THE PREPARATION OF A 3,6-DIHYDRO-2H-1,3,4-OXADIAZIN-2-ONE**

(71) Applicant: Bayer Aktiengesellschaft, 51373 Leverkusen (DE)
(72) Inventor: The designation of the inventor has not yet been filed
(74) Representative: BIP Patents

(57) **Abstract**

The present invention provides methods for the preparation of Compound (I) 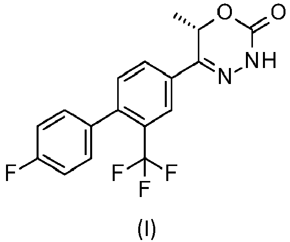 intermediate compounds and their use for the preparation of compound (I).

## Description

The present invention provides a method for the preparation of a 3,6-dihydro-2H-1,3,4-oxadiazin-2-one, novel intermediate compounds and their use in the methods for the preparation of a 3,6-dihydro-2H-1,3,4-oxadiazin-2-one.

### BACKGROUND

Cancer kills over 550,000 people in the United States and over 8 million people world-wide each year. New agents, including small molecules, molecules that impact tissue-specific growth requirements, and immunomodulatory agents, have been shown to benefit a subset of patients whose cancers have unique genomic mutations or other characteristics. Unfortunately, many cancer patients are still left without effective therapeutic options.

One approach to identify new anti-cancer agents is phenotypic screening to discover novel small molecules displaying strong selectivity between cancer cell lines, followed by predictive chemogenomics to identify the cell features associated with drug response. Phenotypic screening identified certain PDE3 inhibitors to be useful for the treatment of certain cancers. In the past those compounds were only known to be used for the treatment of cardiovascular indications (Nature Reviews Drug Discovery 13, 290-314, (2014)). Furthermore as it could be seen that not all PDE3 inhibitors are useful for the treatment of cancer it has been found that only those compounds which stabilize a complex formation between PDE3A and/or PDE3B and SLFN12 cause sell sensitivity and can be used for the treatment of cancer. Especially this has been found for (6S)-5-[4'-fluoro-2-(trifluoromethyl)[biphenyl]-4-yl]-6-methyl-3,6-dihydro-2H-1,3,4-oxadiazin-2-one, known from WO2019/025562. Processes for its production in a lab scale can be found therein, some of them are shown on the following pages, e.g. the synthesis suitable for substituted 3,6-dihydro-2H-1,3,4-oxadiazin-2-ones, including 5-{4-[1-(difluoromethyl)-1H-pyrazol-4-yl]-3-(trifluoromethyl)phenyl}-3,6-dihydro-2H-1,3,4-oxadiazin-2-one, (6S)-5-[4-(2-aminopyridin-4-yl)-3-(trifluoromethyl)phenyl]-6-methyl-3,6-dihydro-2H-1,3,4-oxadiazin-2-one, (6S)-6-methyl-5-{3-(trifluoromethyl)-4-[3-(trifluoromethyl)-1H-pyrazol-1-yl]phenyl}-3,6-dihydro-2H-1,3,4-oxadiazin-2-one, and (6S)-5-[4'-fluoro-2-(trifluoromethyl)[biphenyl]-4-yl]-6-methyl-3,6-dihydro-2H-1,3,4-oxadiazin-2-one is described and is reproduced below exemplarily for (6S)-5-[4'-fluoro-2-(trifluoromethyl)[biphenyl]-4-yl]-6-methyl-3,6-dihydro-2H-1,3,4-oxadiazin-2-one as follows: 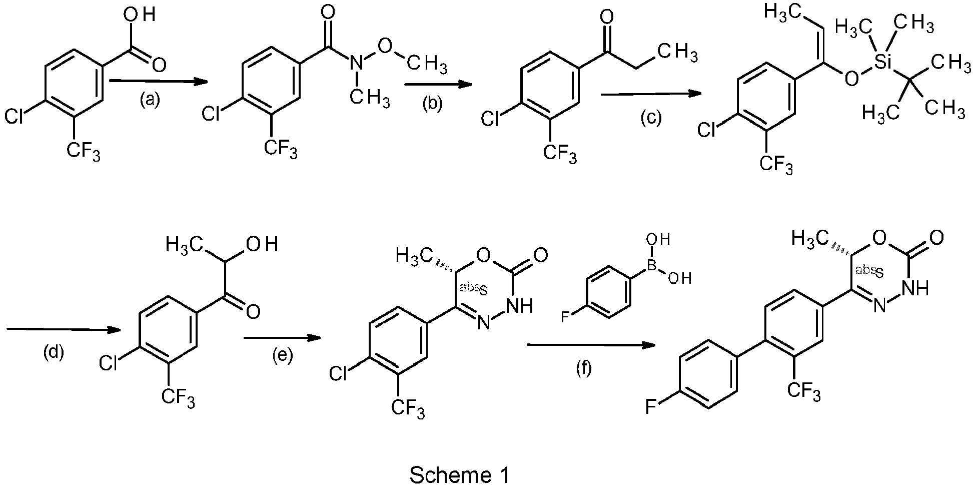
*(a) (i) SOCl₂, (ii) NH(CH₃)(OCH₃), yield 79%*
*(b) CH₃CH₂-Mg-Br, yield 81%*
*(c) (i) Li-hexamethyldisilazane, (ii) tert-butyldimethylchlorosilane, yield 75%*
*(d) AD-mix alpha (Sigma-Aldrich), methanesulfonamide, tert-butanol*/*water, yield 65%*
*(e) H₂N-NHC(O)OCH₃, NaOCH₃, 62% yield*
*(f) PdXPhosG2, K₂CO₃, 1,4-dioxane*/*water, 53% yield, 95% purity, 63%ee for scale-up to [g] portions and 98,0%ee if smaller lab scale amounts were used*

The starting material is commercially available. The following disadvantages prevent from upscaling this synthesis route:
- most of the steps need a column chromatography
- step c has long reaction times and need very low temperature (-78°C)
- the chiral reagent use in step (d) leads not to sufficient enantiomeric purity, at best an ee of 98% were obtained, if small amounts of material are used.
- Furthermore, toxicological and process-related critically Osmium salts are used in this synthesis.
- for step f) a palladium reagent is necessary which is very unfavourable for a last step of an upscaled synthesis as even traces of palladium have to be removed, requiring a tedious work-up using column chromatographies.

Further routes are disclosed in WO2019/025562, some of them are described below very generally to provide an overview only as WO2019/025562 is publically available to derive further details therefrom:

### Synthesis Variant I:

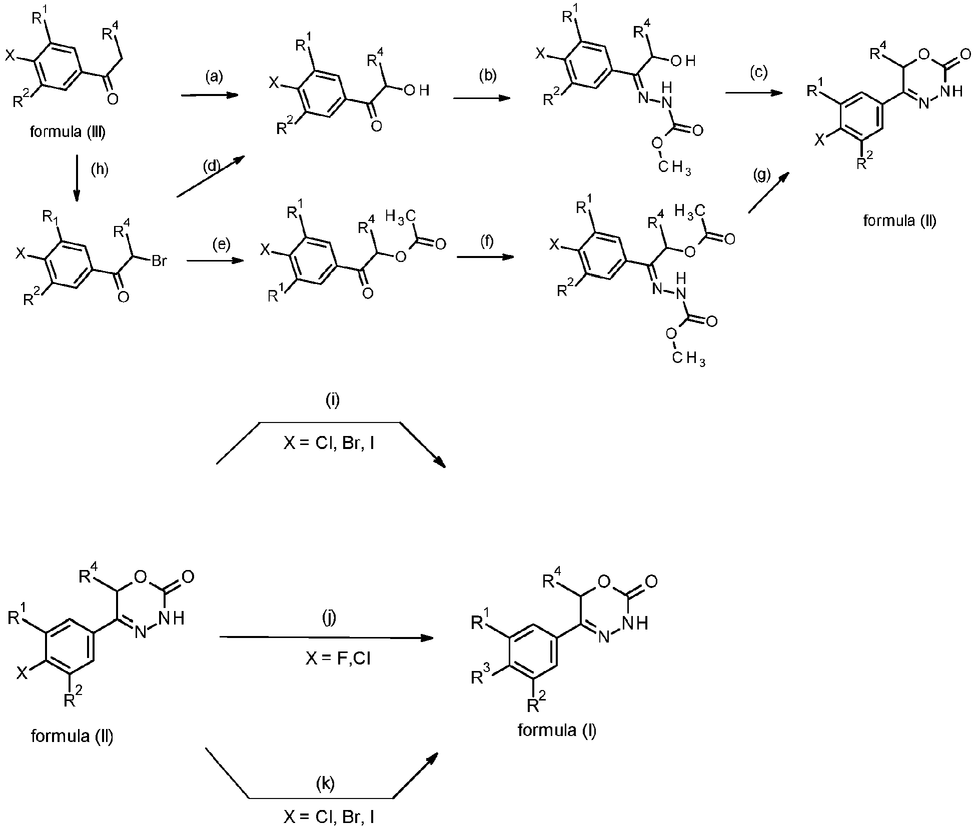

**(a)** Hydroxy(tosyloxy)iodo)benzene, DMSO, water, RT, 18 h; or iodine, DMSO, 60 °C, 18h. For the preparation of the starting material for steps (a) and (h) see e.g. Scheme 3.
**(b)** H₂NNHCOOCH₃, HCI (aq), MeOH, RT, 5h;
**(c)** potassium carbonate, acetonitrile, 60 °C, 18h or NaOEt/EtOH, 0 °C, 10 min, or NaH, EtOH, 0 °C 10 min;
**(d)** sodium formate, sodium hydrogencarbonate, CH₃CN, water, 65°C, 24h;
**(e)** potassium acetate, potassium iodide, 18h, RT;
**(f)** H₂NNHCOOCH₃, HCI (aq), MeOH, RT, 5-18h;
**(g)** potassium carbonate, CH₃CN, 60 °C, 18h or NaOEt/EtOH, 0 °C 10 min, or NaH, EtOH, 0 °C 10 min;
**(h)** Br₂, acetic acid, hydrogen bromide, 18h, room temperature;

Compounds of formula (II) containing chiral centers can be optionally separated by methods known to the person skilled in the art, such as *e.g*. chiral chromatography, to obtain individual enantiomers or diastereomers. This methodology is not efficient for scale-up as half of the product is lost in the last step, the same applies for the next variants (i), (j) and (k) and the chiral chromatography is too cost intensive.
and **(i)** is a Suzuki coupling,
**(j)**, **(k)**: suitable for R³ = NR⁷R⁸, opt. subst. N-linked heterocycloalkyl, partially unsaturated heterocycloalkyl or heteroaryl , again here very unfavourable Pd chemistry is used in the last step.

### Synthesis, variant 2:

The variant 2 is the reaction of a compound of formula (II) with an organo boron compound to achieve at a compound of formula (I), the so called Suzuki coupling reaction. Some alternatives for specific R³ groups are also disclosed which seem not to be of relevance here.

### Synthesis, variant 3:

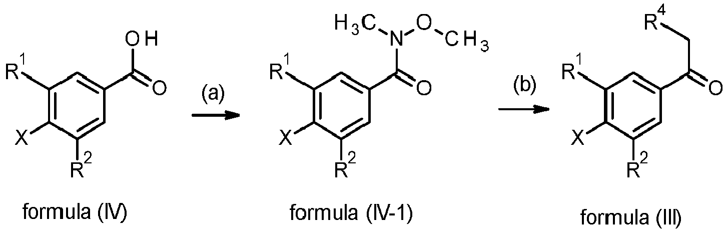

and X is a halogen atom,

### Step(a) (IV) to (IV-1)

A compound of formula (IV) and oxalyl chloride, in a solvent such as e.g. DMF/DCM, are reacted at a temperature range of 0 °C to RT, for 1h-20 h, then HN(OCH₃)CH₃ •HCl, Et₃N/CH₂Cl₂, is added at RT, and the mixture is subsequently reacted for 1h- 3d;

### Step (b) (IV-1) to (III)

A compound (IV-1) and R⁴CH₂MgBr, in which R⁴ is as defined *supra,* in a solvent such as *e.g.* THF, are reacted at 0 °C to RT, for 1h - 20 h, in order to obtain an intermediate compound of formula (III);
and subsequent conversion of intermediate (III) into compounds of general formula (I) by the following steps: 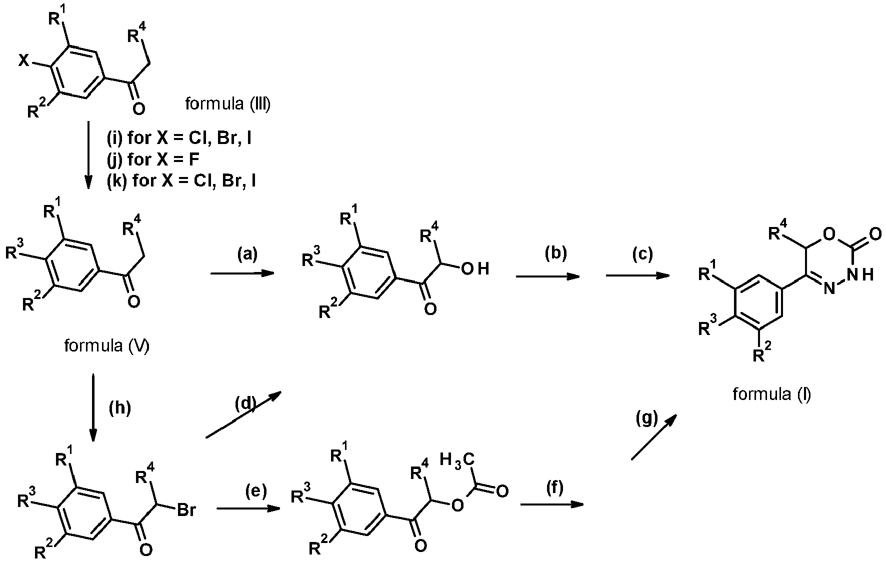 whereby conversion of compound (III) to compound (V) by introduction of R³ again is described (i) via Suzuki coupling reaction, (j) nucleophilic aromatic substitution or (k) by transition metal catalysed amination.

### Step (a)

A compound of formula (V) and Hydroxy(tosyloxy)iodo)benzene in a solvent such as e.g. DMSO, water are reacted at RT, for 18 h; or a compound of formula (V) and iodine, in a solvent such as e.g. DMSO, are reacted at 60 °C, for 18h

### Step (b)

The reaction product of step (a), H₂NNHCOOCH₃, and HCI (aq), are reacted in a solvent such as e.g. MeOH, at RT, for 5h;

### Step (c)

The reaction product of step (b) and potassium carbonate are reacted in a solvent such as e.g. acetonitrile, at 60 °C, for 18h or the reaction product of step (b) and NaOEt/EtOH, are reacted at 0 °C for 10 min, or the reaction product of step (b) and NaH, are reacted in EtOH, at 0 °C for 10 min;

### Step (d)

The reaction product of step (h), sodium formate and sodium hydrogencarbonate, are reacted in a solvent such as e.g. CH₃CN, water, at 65°C, for 24h inorder to obtain the same product as from step (a) which subsequently may be converted to a compound of formula (I) via steps (b) and (c);

### Step (e)

The reaction product of step (h), potassium acetate and potassium iodide are reacted for 18h at RT;

### Step (f)

The reaction product of step (e), H₂NNHCOOCH₃ and HCI (aq), are reacted in a solvent such as *e.g*. MeOH, at RT for 5-18h;

### Step (g)

The reaction product of step (f) and potassium carbonate are reacted in a solvent such as e.g. acetonitrile, at 60 °C, for about 18h or the reaction product of step (f) and NaOEt/EtOH, are reacted at 0 °C for 10 min, or the reaction product of step (f) and NaH, are reacted in a solvent such as e.g. EtOH, at 0 °C for 10 min in order to obtain a compound of formula (I); Enantiomeric separation is conducted on the last step and 50% of the end product is lost which makes this route a very unefficient route.

### Step (h)

The reaction product of step (i, j or k) and a bromination reagent like Br₂/hydrogen bromide are reacted in a solvent such as e.g. acetic acid and water, at room temperature, for 18.

### Synthesis variant 4:

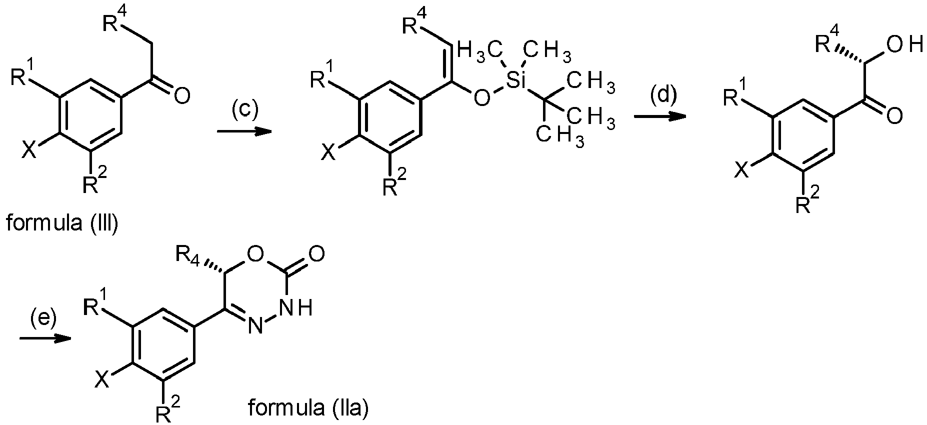

Were X is a halogen atom,
(c) LiHMDS/THF, 1 h, -78 °C, then tBDMSCI (tert-butyldimethylchloro silane), -78 °C to RT, 15h - 2d;
(d) AD-Mix-a, CH3SO2NH2/tBuOH/water, 0 °C to RT 15 h - 2d;
(e) 1. H 2NNHCOOCH3, HCI, MeOH; 2. NaOEt/EtOH or NaOMe/MeOH or NaH/EtOH, chromatography, 97%ee of (IIa);

### Synthesis variant 6 for the preparation of a compound of general formula (II):

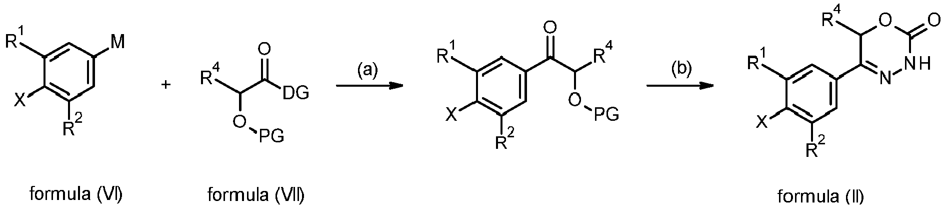

M is a metal-containing group, such as e.g. Li, or MgBr, or MgCI; and X is F, Cl, or Br; and DG is a group displacable from compounds of formula (VII) with reactands of formula (VI), selected from morpholinyl or N(OCH₃)CH₃ (Weinreb amide); and PG is a protecting group suitable for hydroxy groups, e.g. a tri-(C₁C₄-alkyl)-silyl group such as e.g. tert-butyl-dimethylsilyl. Compounds of formulae (VI) and (VII) are known to the person skilled in the art and can be readily prepared from commercially available precursors by known methods.

(a) THF, -20 °C - 20 °C, 1h - 24h, (b) 1. H₂NNHCOOCH₃, HCI, MeOH; 2. TBAF (tetrabutylammonium fluoride), THF; 3. NaOEt/EtOH or NaOMe/MeOH; conversion to a compound of formula (I) can be achieved following Synthesis Variant I, step (i), (j) or (k) mentioned above.

For industrial implementation and the production of larger kilogram amounts, the preparative processes and routes described above are suitable to only a limited extent. Employing suitable building blocks to synthesize (6S)-5-[4'-fluoro-2-(trifluoromethyl)[biphenyl]-4-yl]-6-methyl-3,6-dihydro-2H-1,3,4-oxadiazin-2-one, the most significant disadvantages for upscaling can be summarized as follows over all alternatives:
- all of them require a synthesis step under very low temperature conditions
- in many of them Palladium is used for the last step
- the chiral reagent used is expensive and/or toxic
- all of them require several chromatographic separations, including one chiral separation step
- all of them suffer from low yields

It was therefore desirable to develop a new synthesis, which circumvents these disadvantages and is suitable for large scale production.

Thus, the object of the present invention is to provide a method for preparation of 3,6-dihydro-2H-1,3,4-oxadiazin-2-ones, especially (6S)-5-[4'-fluoro-2-(trifluoromethyl)[biphenyl]-4-yl]-6-methyl-3,6-dihydro-2H-1,3,4-oxadiazin-2-one not suffering from the disadvantages as outlined above which can be worked in industrial scale.

### SUMMARY

It has now been found, and this constitutes a basis of the present invention, that the method for the preparation of the 3,6-dihydro-2H-1,3,4-oxadiazin-2-one below provides higher overall yields, the process can be conducted safely and saves energy as no low temperature step is included and is suitable for a larger scale production.

As a first aspect the invention provides the synthesis of compound (I) comprising the following steps: 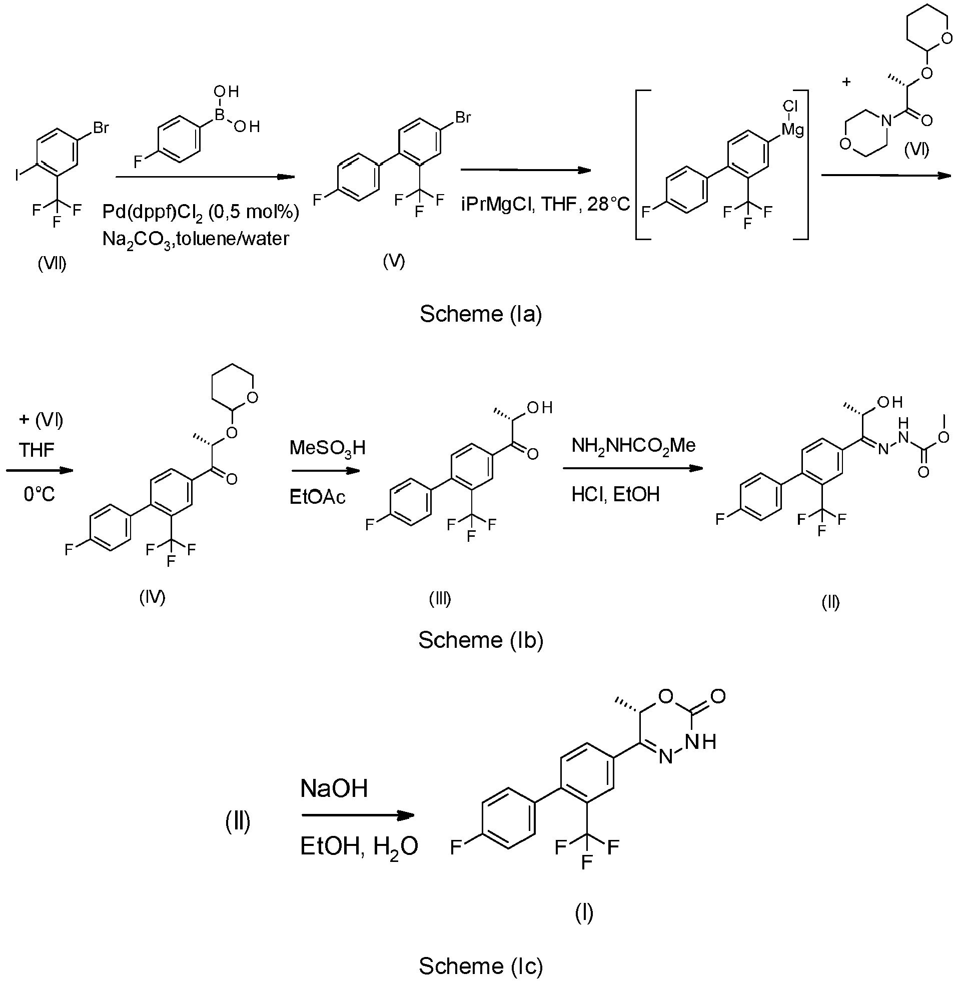 Scheme (Ia-Ic): *Convergent synthesis of the compound of formula (I)*

It is found that this synthesis does not only not suffer from the disadvantages mentioned above but surprisingly in addition delivers the product in a much higher enantiomeric purity and chemical purity (without chromatography in the last synthesis steps) than the synthesis which is provided in WO2019/025562 even though the chiral center is introduced earlier in the synthesis route compared to the WO2019/025562 synthesis.

The main differences to the already published synthesis of (6S)-5-[4'-fluoro-2-(trifluoromethyl)[biphenyl]-4-yl]-6-methyl-3,6-dihydro-2H-1,3,4-oxadiazin-2-one are:
- completely changed reaction sequence (oxadiazinone cyclization in the last step)
- chiral centre from chiral-pool lactic acid, addition of a novel Grignard reagent of the aromatic compound (V) to lactic acid derivative, surprisingly no racemization under the Grignard conditions
- stereoselective hydrazine formation, again surprisingly no racemization under the reaction conditions
- circumvention of low-temperature steps
- critical toxic osmium salts which were contained in the chiral reagent Ad-mix used in the lab synthesis are not used anymore
- palladium-catalyzed cross-coupling is placed at the beginning of the synthesis sequence which now circumvents issues with Palladium-residues that might be difficult to separate from the end product when using Palladium at the end of the synthesis sequence
- use of column chromatography is significantly reduced; instead, destinations and precipitations of intermediates as well as the final product were introduced.
- overall yield over 6 linear steps is increased to 25% yield compared to 10% of the lab synthesis.
- novel synthesis was performed on kg-scale and delivers the product surprisingly in a very high enantiomeric excess (> 99,5 % *ee*) and reproducible high quality.

### DETAILED DESCRIPTION

The synthesis of a compound of formula (I) 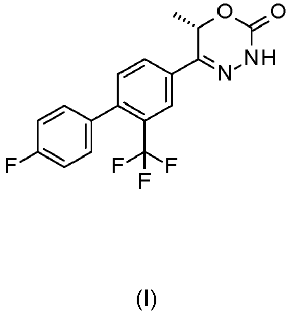 is achieved as shown in scheme (Ia-Ic) above comprising the steps shown therein and as discussed in detail below.

### Synthesis of compound (VI)

### Reaction sequence: (VIII) →(IX) → (VI):

The commercially available compound of formula (VIII) 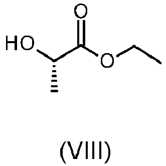 is reacted with morpholine under basic conditions in a protic solvent, especially an alcoholate of an alkali metal (sodium, potassium) in an alcoholic solution, such as e.g. sodiummethanolate in methanol or sodiumethanolate in ethanol,
in order to obtain a compound of formula (IX) 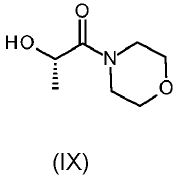 which is further converted into a compound of formula (VI) 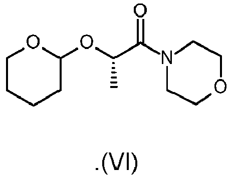 by allowing to react the compound of formula (IX) with dihydropyrane in an aprotic solvent, especially in dichloromethane under acidic catalysis such as e.g. methanesulfonic acid with tetrahydropyrane in order to obtain a compound of formula (VI). The synthesis is carried out analogously to the description in J. Pesti et al., Org. Process Res. Dev. 2009, 13, 716-728; 716-728;

The morpholine group in the reaction step (VIII) → (IX) can be replaced by the Weinreb amide residue -(N(CH₃)OCH₃) by performing a standard amide coupling reacting the acid or acid chloride with the amine, or reacting the ester (VIII) with Cl-Mg-N(CH₃)(OCH₃) under conditions known to the person with ordinary skill.

One aspect of the invention is the use of compound (VI) for the preparation of a compound of formula (I).

Another aspect of the invention is the method of preparing a compound of formula (I) comprising the provision of a compound of formula (VI) by reacting a compound of formula (VIII) with morpholine or the Weinreb amide and further converting the obtained compound of formula (IX) into a compound of formula (VI) or the Weinreb analog by adding the protecting group dihydropyranyl under the conditions as outlined above and which is reacted subsequently in several steps to result in a compound of formula (I).

### Synthesis of compound (V

### Reaction sequence (VII) → (V):

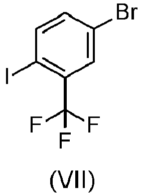

Compound (VII) is reacted with 4-fluorophenyl)boronic acid under palladium catalysis with Pd(dppf)₂Cl₂ [1,1'-Bis(diphenylphosphino)ferrocene]dichloropalladium(II) ) (and addition of cesiumcarbonate or sodiumcarbonate or potassiumcarbonate in a mixture of toluene and water
thereby providing the compound of formula (V)

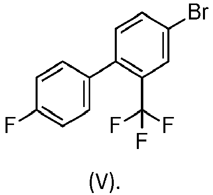

It was surprising that this catalyst was the only one out of the large group of Pd catalysts which provided sufficient selectivity in this reaction.

One aspect of the invention is the compound of formula (V) and its use for the preparation of a compound of formula (I) as well as the use of Pd(dppf)₂Cl₂ [1,1'-Bis(diphenylphosphino)ferrocene]dichloropalladium(II) ) for its preparation.

Further one aspect of the invention is the synthesis of a compound of formula (I) comprising the step (VII) → (V) as outlined above.

### Synthesis of compound (IV)

### Reaction sequence: (V) → (IV)

Compound (V) is converted 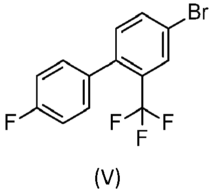 into a Grignard reagent by reacting it with isopropylmagnesiumchloride or ethylmagnesiumchloride or by converting compound (V) into another organometal compound by reacting it with an alternative organometal reagent such as e.g. butyllithium in an aprotic solvent such as tetrahydrofurane (THF) or heptane or a mixture thereof at room temperature or below, for butyllithium at -78°C, and reacting the so obtained Grignard reagent or organometal reagent of the compound of formula (V) subsequently with the compound of formula (VI) 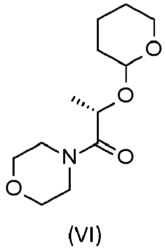 so providing the compound of formula (IV)

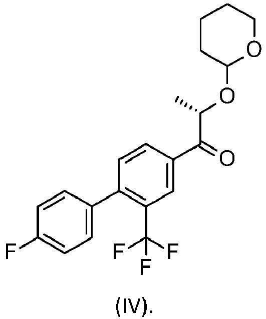

One aspect of the invention is the compound of formula (IV) and its use for the preparation of a compound of formula (I).

Further one aspect of the invention is the synthesis of a compound of formula (I) comprising the step (V) → (IV) as outlined above.

### Synthesis of compound (III)

### Reaction sequence: (IV) → (III):

Allowing an intermediate compound of formula (IV) 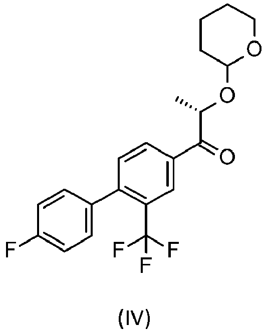 to react under acidic conditions in an aprotic or protic solvent in order to cleave the protecting group to obtain the compound of formula (III)

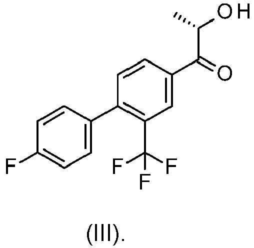

Acidic conditions can be achieved by using a solution of hydrochloric acid, such as e.g. aqueous hydrochloric acid, hydrochloric acid in methanol, hydrochloric acid in acetic acid ethylester, sulfuric acid, p-toluolsulfonic acid, methanesulfonic acid, preferably methanesulfonic acid.

As an aprotic solvent toluol or ethylacetate, preferably ethylacetate can be used and as protic solvent or solvent mixture water and/or methanol or ethanol can be used.

One aspect of the invention is the compound of formula (III) and its use for the preparation of a compound of formula (I).

Further one aspect of the invention is the synthesis of a compound of formula (I) comprising the step (IV) → (III) as outlined above.

### Synthesis of compound (II)

### Reaction sequence: (III) → (II)

The intermediate compound of formula (III) 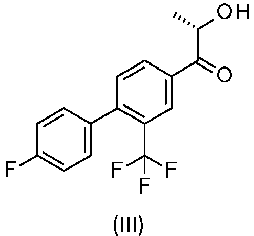 is reacted with NH₂NHCOOCH₃ or NH₂NHCOOCH₂CH₃ under acidic conditions in a suitable solvent to provide the compound of formula (II)

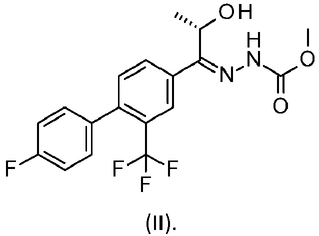

Acidic catalytic conditions are achieved by using methanesulfonic acid, alternatively hydrochloric acid in a suitable solvent such as e.g. water, methanol, ethanol, dioxan, cyclopentylmethylether or acetic acid ethylester or any mixtures thereof. Catalytic amounts of acid may be sufficient to achieve the desired reaction. A catalytic amount can be any amount of less than equimolar, preferably about 0,1mol% of acid relative to the starting material of compound (III) is used, more preferably the ratio as used in the example.

The preferred solvent is acetic acid ethylester.

One aspect of the invention is the compound of formula (II) and its use for the preparation of a compound of formula (I).

Further one aspect of the invention is the synthesis of a compound of formula (I) comprising the step (III) → (II) as outlined above.

### Synthesis of compound (I)

### Reaction sequence: (II) → (I)

The preparation of the compound of formula (I) 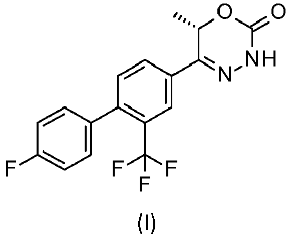 is achieved by allowing a compound of formula (II) 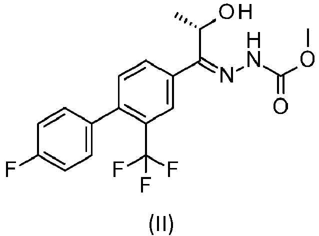 to react under basic conditions in a protic solvent thereby providing the compound of formula (I).

For basic conditions an aqueous sodiumhydroxide - or potassiumhydroxide solution or sodiummethanolate in methanol or sodiumethanolate in ethanol can be used, preferably aqueous sodiumhydroxide solution is used, more preferably 45% sodium hydroxide solution in water.

The protic solvent is methanol or ethanol, preferably ethanol.

Optionally the method above comprises a further a purification step.

Such purification method could be a chromatography, such as e.g. column chromatography or a crystallization.

Some aspects of the invention are the reactions steps as outlined above following the reaction conditions as disclosed in examples 1-5 and their use for the preparation of the compound of formula (I).

As outlined for (6S)-5-[4'-fluoro-2-(trifluoromethyl)[biphenyl]-4-yl]-6-methyl-3,6-dihydro-2H-1,3,4-oxadiazin-2-one above, the corresponding reactions steps can be used to prepare the following compounds: 5-{4-[1-(difluoromethyl)-1H-pyrazol-4-yl]-3-(trifluoromethyl)phenyl}-3,6-dihydro-2H-1,3,4-oxadiazin-2-one, (6S)-5-[4-(2-aminopyridin-4-yl)-3-(trifluoromethyl)phenyl]-6-methyl-3,6-dihydro-2H-1,3,4-oxadiazin-2-one, (6S)-6-methyl-5-{3-(trifluoromethyl)-4-[3-(trifluoromethyl)-1H-pyrazol-1-yl]phenyl}-3,6-dihydro-2H-1,3,4-oxadiazin-2-one, and (6S)-5-[4'-fluoro-2-(trifluoromethyl)[biphenyl]-4-yl]-6-methyl-3,6-dihydro-2H-1,3,4-oxadiazin-2-one by using the appropriate boronic acid compound, e.g. 1-(difluoromethyl)-4-(4,4,5,5-tetramethyl-1,3,2-dioxaborolan-2-yl)-1H-pyrazole, 2-aminopyridine-4-boronic acid pinacol ester, or 4-fluorophenyl boronic acid in order to introduce the ring moiety different from fluorophenyl in the reaction sequence (VII) → (V).

### EXPERIMENTAL SECTION

NMR peak forms are stated as they appear in the spectra, possible higher order effects have not been considered.

Chemical names were generated using the ACD/Name software from ACD/Labs. In some cases generally accepted names of commercially available reagents were used in place of ACD/Name generated names.

The following table 1 lists the abbreviations used in this paragraph and in the Examples section as far as they are not explained within the text body. Other abbreviations have their meanings customary *per se* to the skilled person.

### Table 1: Abbreviations

The following table lists the abbreviations used herein.
- d: doublet (¹H-NMR signal)
- Ad-mix alpha: Reagent, Aldrich, contains (DHQ)2PHAL, Potassium carbonate, powder, Potassium ferricyanide, Potassium osmate dihydrate
- DCM: dichloromethane
- DMSO: dimethylsulfoxide
- dppf: 1,1'-bis(diphenylphosphino)ferrocene
- ee: Enantiomeric excess
- eq.: equivalent(s)
- ESI: electro-spray ionization (MS)
- Et: ethyl
- EtOAc: ethyl acetate
- g: gram
- GCMS: gas chromatography-coupled mass spectroscopy
- h: hour(s)
- ¹H-NMR: proton nuclear magnetic resonance spectroscopy
- HPLC: high performance liquid chromatography
- Hz: hertz
- Kg: kilogram
- L: litre
- LCMS: liquid chromatography-coupled mass spectroscopy
- M: molarity
- m: metre
- m: multiplet (¹H-NMR signal)
- mbar: millibar
- Me: methyl
- MeOH: methanol
- MHz: megahertz
- MS: mass spectrometry
- min: minute(s)
- mL: millilitre
- mm: millimetre
- MS: mass spectroscopy
- m/z: mass-to-charge ratio (MS)
- nm: nanometer
- ppm: parts per million
- Pd(dppf)Cl₂: [1,1'-Bis (diphenylphosphino)ferrocene]palladium (II) dichloride
- q: quartet (¹H-NMR signal)
- quin: quintett (¹H-NMR signal)
- rt: room temperature
- Rₜ: retention time (HPLC)
- s: singlet (¹H-NMR signal)
- sat.: saturated (solution)
- t: triplet (¹H-NMR signal)
- THF: tetrahydrofuran
- UPLC: high performance liquid chromatography
- UV: ultraviolet
- v: volume
- µm: micrometre
- °C: degree celsius

The various aspects of the invention described in this application are illustrated by the following examples which are not meant to limit the invention in any way.

The example testing experiments described herein serve to illustrate the present invention and the invention is not limited to the examples given.

### EXPERIMENTAL SECTION - GENERAL PART

All reagents, for which the synthesis is not described in the experimental part, are either commercially available, or are known compounds or may be formed from known compounds by known methods by a person skilled in the art.

### LCMS (method 1): HSST3

Instrument: Waters ACQUITY SQD UPLC system; column: Waters Acquity UPLC HSS T3 1.8 µm 50 x 1 mm; eluent A: 1 l water + 0.25 ml 99% formic acid, eluent B: 1 l acetonitrile + 0.25 ml 99% formic acid; gradient: 0.0 min 90% A → 1.2 min 5% A → 2.0 min 5% A; flow rate: 0.40 ml/min; UV detection: 208 - 400 nm.

### GCMS (method 2): DSQ-II

Instrument: Thermo Scientific DSQII, Thermo Scientific Trace GC Ultra system; column: Restek RTX-35MS, 15 m x 200 µm x 0.33 µm; constant helium flow: 1.20 ml/min; oven: 60°C; inlet: 220°C; gradient: 60°C, 30°C/min → 300°C (hold time 3.33 min).

### Chiral HPLC (method 3):

System: High performance liquid chromatograph equipped with gradient pumps, UV detector & attached with data recorder and integrator software; column: Chiralpak IA (250*4.6 mm, 5µm); flow: 1 mL/min; column temperature: 40°C; injection volume 10µL, detection 292 nm, run time: 15 min; mobile phase: n-heptane / 2-propanole 90/10 (v/v).

### XRPD

### Method A:

### a) Preparation of the sample

There was no sample preparation.

### b) Measurement details

X-Ray diffraction patterns were recorded at room temperature using XRD-Transmissions-Diffractometer X'Pert PRO (PANalytical) (radiation Cu K alpha 1, wavelength 1.5406 Å, 40mA, 40kV). There was no sample preparation. All X-Ray reflections are quoted as °2Θ (theta) values (peak maxima) with a resolution of ±0.2°.

### Method B

X-ray powder diffraction (XRPD) data were recorded on a STOE STADI P diffractometer using monochromatized CuKa1-radiation, a position sensitive detector, at generator settings of 40 kV and 40 mA. The samples were collected in transition mode, being prepared as a thin layer between two foils. The scanning range was between 2 ° and 40 ° 2 theta with a 0.5° step at 15 sec/step.

### EXPERIMENTAL SECTION - Examples

### Example 1

### 4-Bromo-4'-fluoro-2-(trifluoromethyl)[biphenyl]

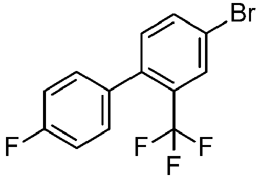

To 166,3 kg toluene and 96,0 kg water were added 24,0 kg (68,4 mol) commercially available 4-bromo-1-iodo-2-(trifluoromethyl)benzene, 12,5 kg (89,3 mol) (4-fluorophenyl)boronic acid and 21,7 kg (204,7 mol) sodium carbonate. To the mixture was added 250 g (0,34 mol) Pd(dppf)Cl₂ and the resulting biphasic solution was heated to 50-60°C and stirred for 6 h. The mixture was cooled down to 20 °C and the phases were separated. The aqueous phase was extracted with 41,6 kg toluene and the toluene phases were combined. The organic phase was filtered through silica gel (12 Kg) to remove Pd-residues. Subsequently, the organic solvent was removed in vacuo to give crude product, which was distilled (1 mbar, 120 °C) to give 16,6 kg 4-bromo-4'-fluoro-2-(trifluoromethyl)[biphenyl] as a colorless liquid in 76% yield.

¹H NMR (600 MHz, DMSO-*d*₆) δ ppm: 7,29 (m, 2 H), 7,36 (t, *J*=7,63 Hz, 3 H), 7,94 (dd, *J₁*=8,22 Hz, *J₂*=1,96 Hz, 1 H), 8,00 (d, *J*=1,96 Hz, 1 H).

GCMS (method 2): Rₜ = 4,69 min; MS m/z = 320, 318 (M-H)⁺, 219, 170, 110.

### Example 2

### (2S)-1-[4'-Fluoro-2-(trifluoromethyl)[biphenyl]-4-yl]-2-(tetrahydro-2H-pyran-2-yloxy)propan-1-one

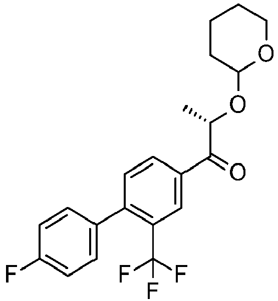

To 1,80 kg (5,64 mol) 4-bromo-4'-fluoro-2-(trifluoromethyl)[biphenyl] were added 1,60 kg THF. At 20 °C, 3,30 kg (6,77 mol) Isopropylmagnesium chloride 2M in THF were added to the above prepared solution over 45 min. During preparation of the Grignard solution, the reaction mixture gets heated to 28°C and the mixture was stirred at 28°C for 5 h. The mixture was cooled to 0-4 °C.

A solution of 1,53 kg (6,28 mol) (2S)-1-(morpholin-4-yl)-2-(tetrahydro-2H-pyran-2-yloxy)propan-1-one prepared according to J. Pesti et al., Org. Process Res. Dev. 2009, 13, 716-728; (using the alternative enantiomer as an ethylester as starting material) in 3,20 kg THF was added to the above prepared Grignard reagent at 0-4 °C within 30 min. The mixture was heated to 20 °C and is stirred for 30 min at 20 °C.

A citric acid solution in water (prepared from 2,70 kg citric acid and 10,10 kg water) was added to the mixture at 20 °C. The addition takes about 60 min.

11,34 kg Ethyl acetate are added to the quenched mixture and the two-phasic mixture was stirred for 10 min at 20 °C. The phases are separated and the lower aqueous phase is discarded.

To the upper organic phase (approx. 24 L) was added 6,50 kg saturated NaCl solution in water and the mixture was stirred for 10 min at 20 °C. The phases were separated and the lower aqueous phase was discarded.

The organic solvent of the upper organic phase (approx. 22,5 L) was distilled off (approx. 17,5 L solvent was distilled off) at 50 °C and 80 mbar to gave an oil. 8,10 kg Ethyl acetate were added to the oil and the solvent was removed at 50 °C and 80 mbar (approx. 9 L solvent was distilled off). To the residue was added 9,10 kg Ethyl acetate.

The prepared solution of (2R)-1-[4'-fluoro-2-(trifluoromethyl)[biphenyl]-4-yl]-2-(tetrahydro-2H-pyran-2-yloxy)propan-1-one in Ethyl acetate (approx. 12 kg solution) was used in the next step (synthesis of (2R)-1-[4'-fluoro-2-(trifluoromethyl)[biphenyl]-4-yl]-2-hydroxypropan-1-one).

¹H NMR (600 MHz, DMSO-*d*₆) δ ppm: 0,99 (m, 1 H), 1,25 (m, 1 H), 1,38 (d, *J*=6,65 Hz, 3 H), 1,44 (d, *J*=6,85 Hz, 3 H), 1,48 (m, 5 H), 1,69 (m, 5 H), 3,47 (m, 1 H), 3,55 (d, *J*=4,89 Hz, 1 H), 3,63 (td, *J₁*=7,43 Hz, *J₂*=7,43 Hz, *J₃*=3,52 Hz, 1 H), 3,81 (m, 1 H), 4,67 (t, *J*=3,33 Hz, 1 H), 4,82 (dd, *J₁*=5,09 Hz, *J₂*=2,15 Hz, 1 H), 5,09 (q, *J*=6,65 Hz, 1 H), 5,24 (q, *J*=7,04 Hz, 1 H), 7,32 (td, *J₁*=8,90 Hz, *J₂*=0,98 Hz, 4 H), 7,42 (m, 4 H), 7,60 (dd, *J₁*=7,92 Hz, *J₂*=4,79 Hz, 2 H), 8,34 (m, 3 H), 8,49 (s, 1 H).

GCMS (method 2): Rₜ = 3,67 min; MS m/z = 240, 219, 201, 170.

### Example 3

### (2S)-1-[4'-Fluoro-2-(trifluoromethyl)[biphenyl]-4-yl]-2-hydroxypropan-1-one

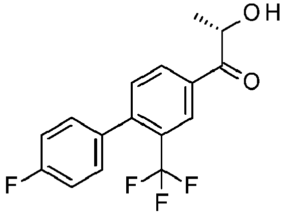

To 12 kg solution of (2S)-1-[4'-fluoro-2-(trifluoromethyl)[biphenyl]-4-yl]-2-(tetrahydro-2H-pyran-2-yloxy)propan-1-one in Ethyl acetate (see example 2) was added 1,09 kg (11,3 mol) of methanesulfonic acid at 20 °C. The mixture was stirred for 2 h at 20 °C.

8 kg water were added to the dark mixture at 20 °C and the mixture was stirred for 10 min. The phases are separated and the aqueous phase (approx. 9 kg) was extracted with 3 kg Ethyl acetate.

The Ethyl acetate phases from both extractions were combined and 6 kg saturated NaCl solution in water was added. The mixture was stirred for 10 min. The phases were separated and the lower aqueous phase was discarded.

The upper organic phase (approx. 16 L) was distilled off at 50 °C and 80 mbar to give an oil. 5 kg Ethyl acetate was added to the oil and the solvent was distilled off at 50 °C and 80 mbar to give an oil.

The resulting oil was distilled (< 0,1 mbar, 100 °C) or can be alternatively purified by flash chromatography (SiO₂) with Heptane/Ethyl acetate (2:1) to give the purified (2S)-1-[4'-fluoro-2-(trifluoromethyl)- [biphenyl]-4-yl]-2-hydroxypropan-1-one (approx. 1,2 kg) as a slightly yellow oil in 68% yield over 2 steps.

¹H NMR (600 MHz, DMSO-d₆) δ ppm: 1,34 (d, *J*=6,85 Hz, 3 H), 5,07 (quin, *J*=6,65 Hz, 1 H), 5,61 (d, *J*=6,46 Hz, 1 H), 7,32 (t, *J*=8,80 Hz, 2 H), 7,41 (dd, *J₁*=8,51 Hz, *J₂*=5,58 Hz, 2 H), 7,58 (d, *J*=8,02 Hz, 1 H), 8,32 (d, *J*=8,02 Hz, 1 H), 8,37 (s, 1 H).

The two steps described here (synthesis of (2S)-1-[4'-fluoro-2-(trifluoromethyl)[biphenyl]-4-yl]-2-(tetrahydro-2H-pyran-2-yloxy)propan-1-one and synthesis of (2S)-1-[4'-fluoro-2-(trifluoromethyl)[biphenyl]-4-yl]-2-hydroxypropan-1-one) were performed several times and batches were combined to get 3,05 kg of (2S)-1-[4'-fluoro-2-(trifluoromethyl)[biphenyl]-4-yl]-2-hydroxypropan-1-one to perform the next step (Example 4) in reasonable batch size.

### Example 4

### Methyl (2Z)-2-{(2S)-1-[4'-fluoro-2-(trifluoromethyl)[biphenyl]-4-yl]-2-hydroxypropylidene} hydrazinecarboxylate

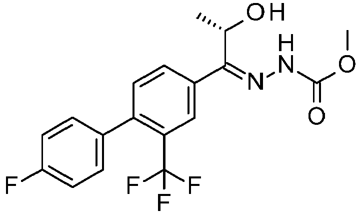

To 3,05 kg (9,76 mol) (2S)-1-[4'-fluoro-2-(trifluoromethyl)[biphenyl]-4-yl]-2-hydroxypropan-1-one was added 21,2 kg ethanol. 0,88 kg (9,76 mol) methyl hydrazine carboxylate as well as 0,1 L of HCI 0,1M in water (0,01 mol) were added at 20 °C. The mixture was heated to reflux for 18 h.

Approx. 20 L of solvent were distilled off at 90 °C and 7,4 kg Diisopropyl ether was added. Approx. 9 L of solvent were distilled off at 80-90 °C and 7,4 kg Diisopropyl ether was added. Approx. 12 L of solvent were distilled off at 80-90 °C and 7,4 kg Diisopropyl ether was added. The mixture was cooled to 2 °C over 60 min and was stirred for approx. 8 h at 2 °C.

The precipitated product was filtered and washed twice with each time 2 kg cold Diisopropyl ether.

The wet product was dried at 50 °C in-vacuo to give 2,01 kg of methyl (2Z)-2-{(2S)-1-[4'-fluoro-2-(trifluoromethyl)[biphenyl]-4-yl]-2-hydroxypropylidene}hydrazinecarboxylate as a white solid in 54% yield.

¹H NMR (400 MHz, DMSO-*d*₆) δ ppm: 1,38 (d, *J*=6,72 Hz, 3 H), 3,72 (s, 3 H), 5,27 (dd, *J₁*=6,79 Hz, *J₂*=3,36 Hz, 1 H), 6,38 (d, *J*=3,06 Hz, 1 H), 7,36 (m, 5 H), 8,00 (d, *J*=8,34 Hz, 1 H), 8,12 (s, 1 H), 11,06 (s, 1 H).

LCMS (method 1): Rₜ = 1,04 min; MS (ESIpos): m/z = 385 (M+H)⁺

### Example 5

### (6S)-5-[4'-Fluoro-2-(trifluoromethyl)[biphenyl]-4-yl]-6-methyl-3,6-dihydro-2H-1,3,4-oxadiazin-2-one

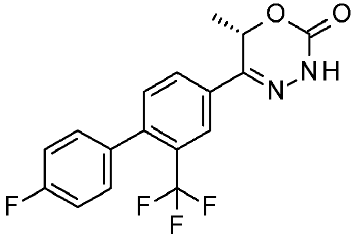

To 15,8 kg ethanol was added 2,00 kg (5,20 mol) methyl (2Z)-2-{(2S)-1-[4'-fluoro-2-(trifluoromethyl) [biphenyl]-4-yl]-2-hydroxypropylidene}hydrazinecarboxylate at 20 °C. 464 g sodium hydroxide solution (45% in water) was added and the mixture was stirred at 20 °C for 1 h. Subsequently, 312 g acetic acid was added and the mixture was stirred at 20 °C for 30 min.

After filtration (final processing), 21,0 kg purified water was added to the mixture at 20°C. A suspension of seed crystals*) (modification C) in ethanol/water (1:1) was added. The mixture was stirred for at 20 °C for 8 h. The crystallized product was filtered, washed twice each time with 2 L water/ethanol (1:1) and dried at 50°C in-vacuo to give 1,68 kg (6S)-5-[4'-fluoro-2-(trifluoromethyl)[biphenyl]-4-yl]-6-methyl-3,6-dihydro-2H-1,3,4-oxadiazin-2-one as a white solid in 92% yield.

### *) seed crystals were produced by the following method:

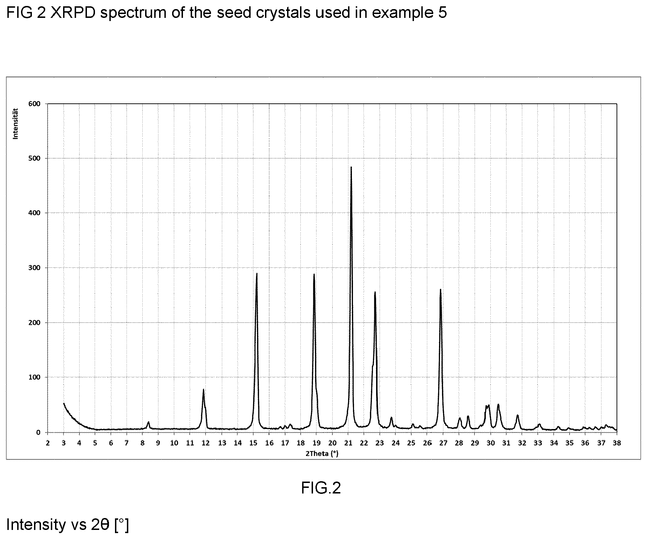

0,5 g of (6S)-5-[4'-fluoro-2-(trifluoromethyl)[biphenyl]-4-yl]-6-methyl-3,6-dihydro-2H-1,3,4-oxadiazin-2-one (obtained as modification A and B in analogy to e.g. published methods) were added to 10 mL of a 1:1 mixture of water/ethanol (approx.. 5 w.%). A suspension of (6S)-5-[4'-fluoro-2-(trifluoromethyl)[biphenyl]-4-yl]-6-methyl-3,6-dihydro-2H-1,3,4-oxadiazin-2-one in ethanol/water (1:1) was formed. After stirring the suspension for 16 h at 30 °C, the suspension was filtered. The solid was dried to obtain (6S)-5-[4'-fluoro-2-(trifluoromethyl)[biphenyl]-4-yl]-6-methyl-3,6-dihydro-2H-1,3,4-oxadiazin-2-one in modification C as shown in FIG. 2, obtained by method B.

¹H NMR (500 MHz, DMSO-*d*₆) δ ppm: 1,49 (d, *J*=6,94 Hz, 3 H), 5,96 (q, *J*=6,91 Hz,1 H), 7,31 (t, *J*=7,96 Hz, 2 H), 7,40 (t, *J*=6,48 Hz, 2 H), 7,53 (d, *J*=8,04 Hz, 1 H), 8,06 (dd, *J₁*=8,12 Hz, *J₂*=1,50 Hz, 1 H), 8,17 (s, 1 H), 11,32 (s, 1 H).

LCMS (method 1): Rₜ = 1,02 min; MS (ESIneg): m/z = 351 (M-H)⁻

Chiral HPLC (method 3): Rₜ (R-enantiomer) = 10,5 min; Rₜ (S-enantiomer) = 11,5 min. ee (S-enantiomer) > 99,5%.

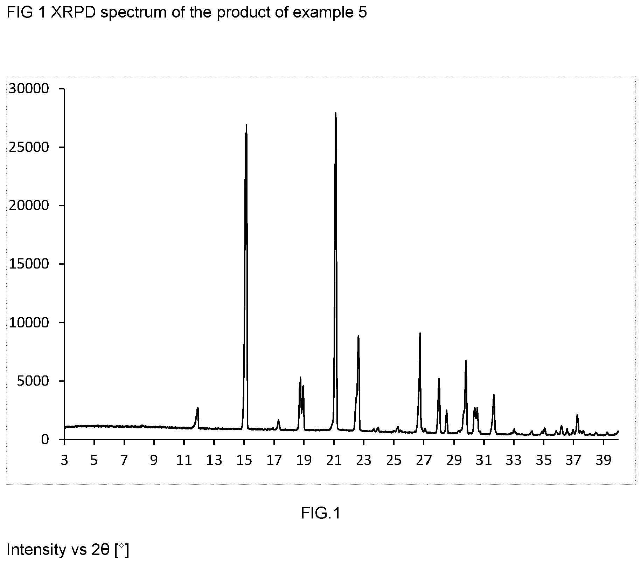

The XRPD of Modification C as obtained by example 1.5 is illustrated by FIG. 1 obtained using method B.

Subsequently optionally a micronization step could be performed.

In analogy to Example 5, five batches were prepared and subsequently combined and micronized twice in a row to give 5,60 kg of micronized material. Here, details for micronization are given:
Five batches of (6S)-5-[4'-fluoro-2-(trifluoromethyl)[biphenyl]-4-yl]-6-methyl-3,6-dihydro-2H-1,3,4-oxadiazin-2-one were prepared according to Example 5 and were combined. The combined batches were micronized twice in a row using an air jet mill.

### a) Used Device:

| Used Device | Mill | 200 mm spiral jet mill (Bayer design) |
|---|---|---|
| | Dosinq device (Type) | vibrating chute |
| | Grinding chamber lining (material) | PTFE |
| | Injector motive nozzle (diameter) | 2,9 mm |
| | Grindinq nozzle (diameter) | 2,2 mm 32° |
| | Collectinq nozzle (diameter) | 8 mm PTFE |
| | Outlet opening (diameter) | 28,3 mm PTFE |

### b) Grinding Parameters

| **Grinding Parameter** | | | **Actual value** |
|---|---|---|---|
| | Injector air pressure | [bar] | 4,3 |
| | Grindinq air pressure | [bar] | 6 |
| | Product throughput | [g/min] | 100 |
| | Total grinding time | [min] | 68 |

### c) The micronized product obtained has the following properties:

Particle Size distribution (X10/X50/X90): 1,2/4/10 µm.

### The instrumental set-up for particle size analysis:

Laser diffraction pattern analyzer (Sympatec HELOS & RODOS), R3:0.5/0.9.... 175µm), (100 mm focal length).
Dry dispersion (RODOS) at 4 bar,
Mathematical model (LD) - Sympatec Code, basis Fraunhofer Diffraction

### Example 6 - lab synthesis [g] scale for comparison following the route as disclosed in example 135 of WO2019/025562, last step:

### The synthesis of (6S)-5-[4'-Fluoro-2-(trifluoromethyl)[biphenyl]-4-yl]-6-methyl-3,6-dihydro-2H-1,3,4-oxadiazin-2-one

In a vessel was charged (6S)-5-[4-chloro-3-(trifluoromethyl)phenyl]-6-methyl-3,6-dihydro-2H-1,3,4-oxadiazin-2-one (7.65 g, 25.10 mmol), 4-fluorophenylboronic acid (5.27 mg, 37.64 mmol) ,2-(Dicyclohexylphosphino)-2',4',6'-triisopropylbiphenyl (XPhos, 718 mg, 1.51 mmol) and K₂CO₃ (7.18 g, 50.19 mmol) and Chloro(2-dicyclohexylphosphino-2',4',6'-triisopropyl-1,1'-biphenyl)[2-(2'-amino-1,1'-biphenyl)]palladium(II), X-Phos aminobiphenyl palladium chloride precatalyst (XPhos-Pd-G2, 1.18 g, 0.75 mmol) in 1,4-dioxane (115 mL) and water (38.4 mL). The resultant mixture was degassed via nitrogen-filled balloon for 5 mins, with subsequent heating at 80 °C for 2 hours. After this time, the mixture was diluted with Ethyl acetate (50 mL) and washed with saturated aqueous NaCl solution (3 x 20 mL), dried (Na₂SO₄), filtered and concentrated at reduced pressure. The residue was purified via Biotage Isolera chromatography (using a gradient of eluents; 1:0 to 1:1 Heptane: Ethyl acetate). The desired fractions were combined, concentrated in vacuo and triturated with tert-butyl methyl ether/Heptane (1:1) with the insoluble material isolated by suction filtration and dried in vacuo to give a grey powder (6.45 g, 95.88 %ee). This material was suspended in 40%Ethanol/Water and warmed to facilitate dissolution. Once the majority of the material was dissolved, the solution was filtered through a cotton plug and allowed to cool to room temperature and stand at room temperature for 60 h. After this time, the crystallized material was isolated by suction filtration and dried in vacuo to afford the product as a colourless crystalline solid (4.48 g, 89.1 %ee). This material was suspended in 40%Ethanol/Water and warmed to facilitate dissolution, with the solution was filtered through a cotton plug and allowed to cool to room temperature and stand at room temperature for 16 h, with the crop of crystalline material isolated to afford (6S)-5-[4'-fluoro-2-(trifluoromethyl)[biphenyl]-4-yl]-6-methyl-3,6-dihydro-2H-1,3,4-oxadiazin-2-one in 39% yield (3.46 g, 93.78 %ee)

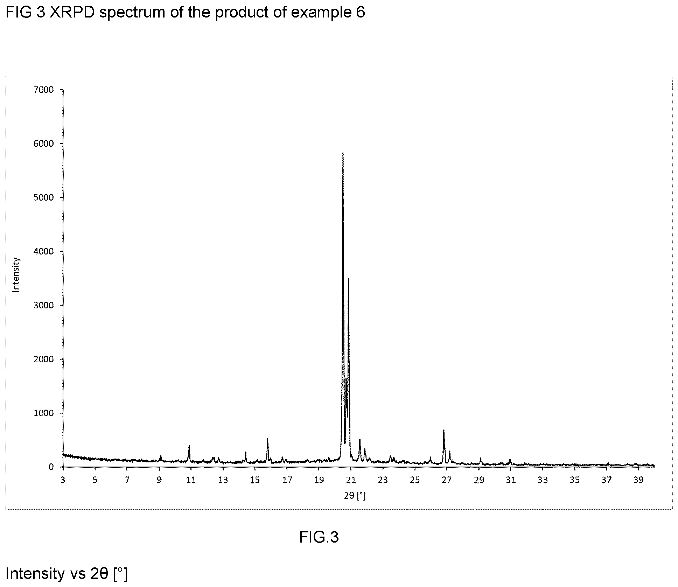

The XRPD of Modification B as obtained is illustrated in FIG. 3 obtained by method A.

### Example 7 - recrystallization of compound (I)

In a flask (6S)-5-[4'-fluoro-2-(trifluoromethyl)[biphenyl]-4-yl]-6-methyl-3,6-dihydro-2H-1,3,4-oxadiazin-2-one, 2.00 g, (93.78 %ee) of the product obtained in example 6 which was stored a room temperature for about 7 month, was suspended in 20 mL of water and 20 mL of ethanol and stirred at room temperature for 30 min. The solid was collected by filtration and the collected solids were again suspended in 20 mL of water and 20 mL of ethanol and stirred at room temperature for 30 min. The remaining crystalline solid was collected by filtration again, dried in a vacuum oven at 50°C overnight to yield 1.62g of the title compound (6S)-5-[4'-fluoro-2-(trifluoromethyl)[biphenyl]-4-yl]-6-methyl-3,6-dihydro-2H-1,3,4-oxadiazin-2-one with 99.7% ee (99% purity by LCMS and H-NMR).

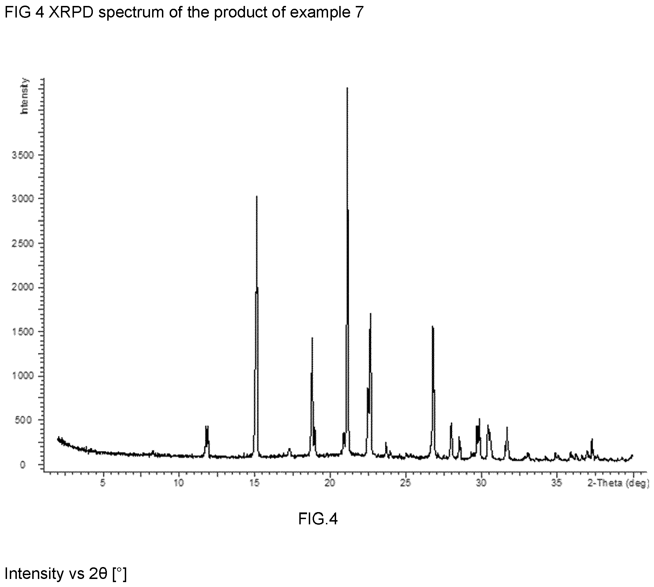

The XRPD of the product obtained is shown in FIG. 4 obtained by method A, which is modification C. Its reflections are shown in the following table:

| Reflections [°2Theta] |
|---|
| 8.3 |
| 11.8 |
| 11.9 |
| 15.1 |
| 15.1 |
| 17.3 |
| 18.8 |
| 18.9 |
| 20.9 |
| 21.1 |
| 22.5 |
| 22.6 |
| 23.6 |
| 26.8 |
| 28.0 |
| 28.5 |
| 29.6 |
| 29.8 |
| 30.4 |
| 30.5 |
| 31.5 |
| 31.6 |
| 33.0 |
| 34.9 |
| 35.1 |
| 35.8 |
| 36.2 |
| 36.6 |
| 36.9 |
| 37.3 |
| 37.6 |

## Claims

1. A method for the preparation of a compound of formula (I) 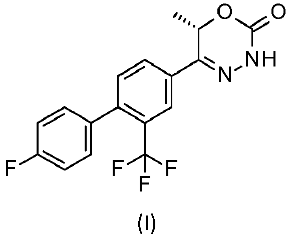 comprising the step of allowing a compound of formula (II) 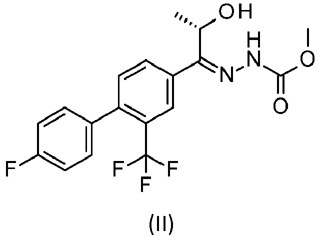 to react under basic conditions in a protic solvent thereby providing the compound of formula (I).

2. The method of claim 1, where the basic condition is an aqueous sodiumhydroxide - or potassiumhydroxide solution or sodiummethanolate in methanol or sodiumethanolate in ethanol.

3. The method of claim 1 further comprising a purification step.

4. The method of claim 1, where the protic solvent is methanol or ethanol.

5. The method of claim 1, further comprising the step of allowing an intermediate compound of formula (III) 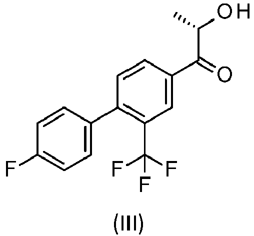 to react with NH₂NHCOOCH₃ or NH₂NHCOOCH₂CH₃ under acidic conditions in a suitable solvent to provide the compound of formula (II)

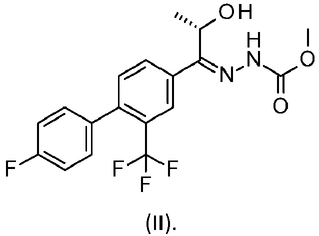

6. The method of claim 5 where acidic conditions are achieved by addition of a solution of hydrochloric acid in a suitable solvent selected from water, methanol, dioxan, cyclopentylmethylether or acetic acid ethylester or any mixtures thereof.

7. The method of claim 6 where the amount of hydrochloric acid is catalytic.

8. The method of claims 1 and 5, further comprising the step of allowing an intermediate compound of formula (IV) 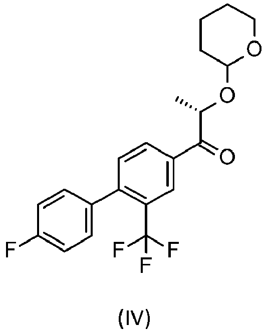 to react under acidic conditions in an aprotic or protic solvent in order to cleave the protecting group to achieve the compound of formula (III)

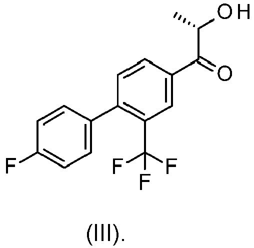

9. The method according to claim 8 where aqueous hydrochloric acid, hydrochloric acid in methanol, hydrochloric acid in acetic acid ethylester, sulfuric acid, p-toluolsulfonic acid, or methanesulfonic acid is used to achieve the acidic conditions.

10. The method according to claim 8 where acetic acid ethylester or toluol is used as aprotic solvent or water and/or methanol or ethanol is used as protic solvent or mixture of solvents.

11. The method of claims 1, 5 or 8, further comprising the step of converting the compound of formula (V) 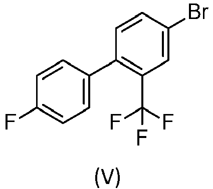 into an organometal reagent by reacting it with isopropylmagnesiumchloride in an aprotic solvent and reacting the so obtained organometal reagent of compound of formula (V) subsequently with the compound of formula (VI) 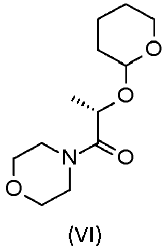 so providing the compound of formula (IV)

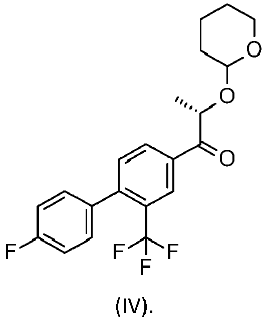

12. The method according to claim 11, where the aprotic solvent is tetrahydrofurane or a mixture of tetrahydrofurane with heptane.

13. The method according to claim 11, where the compound forming the organometal reagent reacting with the compound of formula (V) is ethylmagnesiumchloride or Butyllithium.

14. The method of claims 1, 5, 8, or 11, further comprising the step of reacting the compound (VII) 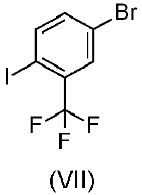 with (4-fluorophenyl)boronic acid under palladium catalysis and addition of sodiumcarbonate, potassiumcarbonate or cesiumcarbonate in a mixture of toluene and water
thereby providing the compound of formula (V)

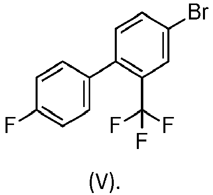

15. The method according to claim 14 where the palladium catalyst is [1,1'-Bis (diphenylphosphino)ferrocene]palladium (II) dichloride.

16. The intermediate compound (V) according to claim 13

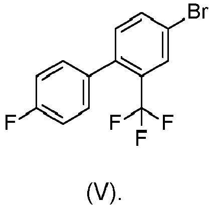

17. The intermediate compound (IV) 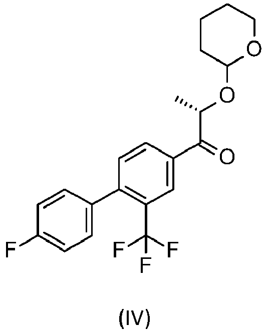 according to claim 11.

18. The intermediate compound (III) 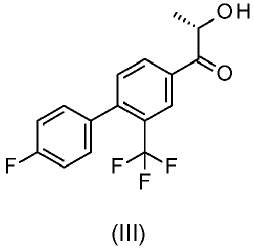 according to claim 8.

19. The intermediate compound (II) 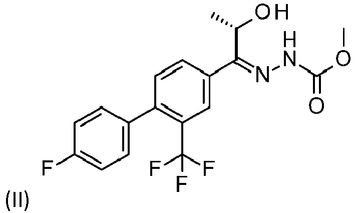 according to claim 5.

20. Use of intermediate compounds (II), (III), (IV), (V) and (VI) for the preparation of compound (I).
